## Europäisches Patentamt
## European Patent Office
## Office européen des brevets

(19)

(11) Numéro de publication: **0 154 593**
**A1**

(12)
# DEMANDE DE BREVET EUROPEEN

(21) Numéro de dépôt: **85420021.9**

(22) Date de dépôt: **08.02.85**

(51) Int. Cl.⁴: **A 61 M 5/20**
**A 61 M 5/31**

(30) Priorité: **13.02.84 FR 8402620**

(43) Date de publication de la demande:
**11.09.85 Bulletin 85/37**

(84) Etats contractants désignés:
**AT BE CH DE FR GB IT LI LU NL SE**

(71) Demandeur: **Laboratoire AGUETTANT**
**1, avenue Jules Carteret**
**F-69007 Lyon(FR)**

(72) Inventeur: **Brunet, Jean-Louis**
**Clinique Charcot 51-53 rue du Commandant Charcot**
**F-69110 Sainte-Foy-Les-Lyon(FR)**

(54) **Seringue à usage medical.**

(57) L'invention a pour objet la modification d'une seringue automatique permettant un conditionnement et une stérilisation simplifiée du système existant grâce à une bague d'adaptation (6) solidarisant l'étui injecteur (2) et l'étui déclancheur (1) permettant l'interposition d'une membrane perforable (5), assurant l'étanchéité de l'étui injecteur et ainsi l'inviolabilité et le maintien de la stérilité de l'ensemble ampoule aiguille contenu à l'intérieur.

Le système de bague par une longueur variable (10, 11) de cette bague, permet par ailleurs d'injecter des doses différentes du médicament contenu dans l'ampoule.

FIGURE 4

EP 0 154 593 A1

1

## SERINGUE A USAGE MEDICAL

La présente invention se rapporte à une seringue automatique à usage médical du type de celle décrite dans la demande de brevet du 17 Janvier 1983 n° 8300891 pour seringue à usage médical.

Le but de la présente invention est de permettre un conditionnement avec stérilisation simplifiée du système et un rechargement facile pour les utilisateurs après usage.

La modification amenée permet par ailleurs de concevoir par deux présentations analogues très simples l'injection de la moitié ou de la dose totale du médicament contenu dans la seringue.

Dans la description du brevet du 17 Janvier 1983,la seringue automatique,comme le montre la figure 1,comporte un étui déclancheur 1 et un étui injecteur 2 dans lequel est placé une ampoule mobile 3.Cet ensemble étant dissociable par simple dévissage,si l'utilisateur sépare l'étui injecteur de l'étui déclancheur,l'ampoule mobile se trouve en contact avec l'air ambiant et la stérilité de l'aiguille 8 n'est plus maintenue d'autant que l'utilisateur peut retirer l'ampoule de l'étui injecteur sans difficulté.D'autre part,même si le système n'est pas ouvert,sa conception fait qu'à l'évidence,il n'est pas parfaitement-étanche,avec des zones de perméabilité en particulier à l'endroit où les parties 1 et 2 se vissent et surtout au niveau de la goupille de sécurité 4.

Cet ensemble ne peut être en fait maintenu stérile que s'il est enfermé après stérilisation dans un emballage étanche,ce qui est un inconvénient lors de l'utilisation car celà retarde d'autant la mise en oeuvre de la seringue lors d'un état d'urgence.

Enfin,la stérilisation de ce système impose des opérations compliquées étant donné qu'il est nécessaire de stériliser l'ensemble.

L'invention a pour objet,en conservant l'avantage du dispositif,de permettre une stérilisation facile,d'assurer l'inviolabilité de l'ampoule et de l'aiguille,de permettre un rechargement facile après utilisation.

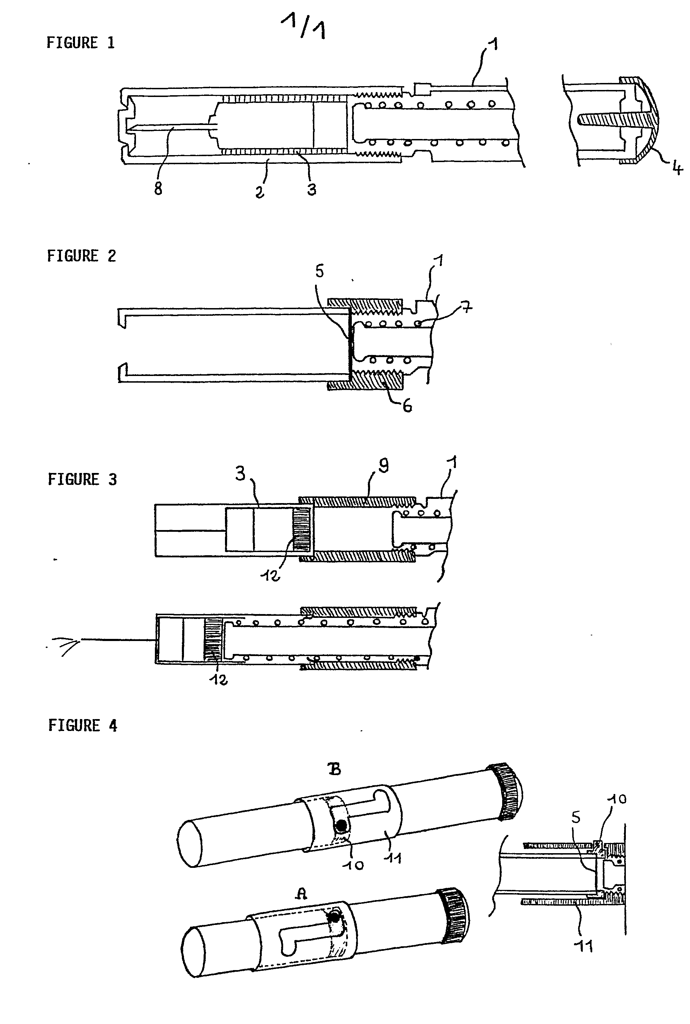

Dans une forme de réalisation(figure 2),l'étui injecteur 2 selon l'invention est rendu étanche par une membrane perforable 5,en caoutchouc ou en plastique par exemple,maintenu en place par une bague d'adaptation 6 sur laquelle vient se visser l'étui déclancheur 1.Cette membrane est bien sûr prévue pour être traversée par le ressort propulseur 7 sans que le fonctionnement de celui-ci puisse être gêné d'aucune façon que ce soit,

2

et en particulier sans perte de puissance.

L'ensemble étui injecteur-ampoule mobile devient donc indissociable, stérilisable globalement,assurant le maintien de la stérilité et l'inviolabilité de l'ampoule 3 et de l'aiguille 8,et permettant par ailleurs la manipulation directe de la seringue sans aucune protection supplémentaire,en particulier pas d'emballage étanche à ouvrir au préalable dans le cas d'une utilisation urgente.

Enfin,il n'est plus nécessaire de stériliser l'étui déclancheur et ses différents composants,le rechargement étant donc par ailleurs très simplifié puisqu'il suffit de recomprimer le ressort 7 avant de revisser un étui "injecteur-recharge".

En ce qui concerne la bague d'adaptation 6,l'allongement de celle-ci, comme celà est montré à la figure 3,fait que lorsque le ressort s'est complètement détendu,il n'a pu enfoncer le bouchon piston 12 qu'à la moitié de l'ampoule 3,ne délivrant par conséquent que la moitié de la dose du médicament contenu dans l'ampoule.Ceci est réalisable grâce à une bague différente 9 de longeur calculée.Celà conduit à devoir utiliser deux sortes de recharges(bague courte ou bague longue).Mais on peut aussi utiliser une bague de longueur variable telle que la figure 4 le montre. Cette bague est constituée de deux pièces 10 et 11 mobiles l'une sur l'autre avec deux positions verrouillables A et B permettant pour la première l'injection d'une dose complète et pour la seconde l'injection d'une demi dose.Ceci permet très avantageusement une adaptation de la posologie du médicament délivré en fonction de l'indication médicale.

Comme il ressort de ce qui précède,l'invention apporte une grande amélioration à la technique existante en fournissant une seringue destinée aux autoinjections,dont la structure est telle que le conditionnement avec surtout la stérilisation en sont facilités.D'autre part, l'utilisation en est simplifiée et peut s'adapter selon les cas par délivrance de doses partielles ou totales du médicament contenu.

Comme il va de soi,l'invention ne se limite pas aux seules formes de d'exécution décrites ci dessus,mais embrasse au contraire toutes les variantes de réalisation.

REVENDICATIONS

1/ seringue à usage médical du type,caractérisée par le fait qu'elle comporte une bague d'adaptation 6 solidarisant l'étui déclancheur 1 et l'étui injecteur 2 et permettant la fixation d'une membrane perforable 5 à la partie supérieure de l'étui injecteur.

2/ Seringue médicale selon la revendication précédente caractérisée en ce que des bagues d'adaptation de différentes longueurs peuvent être utilisées.

3/ Seringue selon l'une quelconque des revendications précédentes caractérisée en ce que la bague d'adaptation est constituée de deux pièces 10 et 11 mobiles l'une sur l'autre.

FIGURE 1

1

8

8    2    3

4

FIGURE 2

5    1

7

6

FIGURE 3

3    9    1

12

12

FIGURE 4

B

10    11

10

5    10

A

11

11

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| Y | US-A-3 396 726  (SARNOFF) <br> * Figure 2 * | 1 | A 61 M   5/20 <br> A 61 M   5/31 |
| | --- | | |
| Y | AU-A-  499 922  (VOGELMAN et al.) <br> *  Page 7, lignes 28-30; figure 5 * | 1 | |
| | --- | | |
| A | US-A-3 612 051  (R. OLVERA ARCE) <br> *  Colonne  3, lignes 27-36; figures 3,4 * | 1 | |
| | --- | | |
| A | FR-A-2 040 753  (STEINER) <br> *  Page  2, ligne  36  - page 3, ligne 4; figures * | 1 | |
| | --- | | DOMAINES TECHNIQUES RECHERCHES (Int. Cl.4) |
| A | FR-A-1 320 820  (STEINER) <br> * Figures 3,4, repère 24 * | 1,2 | A .61 M |
| | --- | | |
| A | FR-A-2 089 176  (BECTON DICKINSON & CO.) <br> * Page 3, lignes 23-26; figures * | 1 | |
| | ----- | | |

Le présent rapport de recherche a été établi pour toutes les revendications

| Lieu de la recherche <br> LA HAYE | Date d'achèvement de la recherche <br> 08-05-1985 | Examinateur <br> GLAS J. |
|---|---|---|